**Europäisches Patentamt**

**European Patent Office**

**Office européen des brevets**

(19)

(11) Veröffentlichungsnummer: **0 253 185**
**A1**

(12) # EUROPÄISCHE PATENTANMELDUNG

(21) Anmeldenummer: **87109284.7**

(22) Anmeldetag: **27.06.87**

(51) Int. Cl.4: **A61B 5/10** , **G01B 11/24** , **G01C 11/00**

(30) Priorität: **30.06.86 DE 3621927**

(43) Veröffentlichungstag der Anmeldung:
**20.01.88 Patentblatt 88/03**

(84) Benannte Vertragsstaaten:
**AT BE CH DE ES FR GB GR IT LI LU NL SE**

(71) Anmelder: **Landwehr, Ulrich M.**
**Bahnhofstrasse 8**
**D-3000 Hannover 1(DE)**

(72) Erfinder: **Landwehr, Ulrich M.**
**Bahnhofstrasse 8**
**D-3000 Hannover 1(DE)**

(74) Vertreter: **Döring, Roger, Dipl.-Ing.**
**Kabelkamp 20**
**D-3000 Hannover 1(DE)**

(54) **Verfahren und Vorrichtung zur Ermittlung der Abmessungen eines Gegenstandes.**

(57) Bei einem Verfahren zur Ermittlung der Abmessungen eines Gegenstandes (4) auf photographischem Wege, insbesondere der menschlichen Körpermaße, wird eine Photographie des Gegenstandes (4) zusammen mit einem Meßraster (6) hergestellt und während der Aufnahme ein schräg von oben einfallendes Muster aus horizontal verlaufenden Linien auf den Gegenstand projiziert. Muster wird mittels einer Vielzahl von Lichtquellen (2a-2e) erzeugt, deren optische Achse unter einem Winkel von nahezu 45° zur Vertikalen verlaufen

Fig 1

EP 0 253 185 A1

## Verfahren und Vorrichtung zur Ermittlung der Abmessungen eines Gegenstandes

Die Erfindung betrifft ein Verfahren zur Ermittlung der Abmessungen eines Gegenstandes auf photographischem Wege, insbesondere der menschlichen Körpermaße, bei dem eine Photographie des Gegenstandes zusammen mit einem Meßraster hergestellt und während der Aufnahme ein schräg von oben einfallendes Muster aus horizontal verlaufenden Linien auf den Gegenstand projiziert wird.

Ein solches Verfahren ist zum Beispiel aus der DE-PS 29 48 010 sowie der DE-OS 34 25 913 bekannt. Das schräg von oben einfallende Linienmuster ermöglicht dabei die Ermittlung der dritten Dimension. Weichen zum Beispiel die auf den Gegenstand projizierten Linien nach oben aus der Horizontalen ab, so befindet sich an dieser Stelle eine Erhebung. Ist das Linienmuster eng genug, läßt sich ein sehr genaues dreidimensionales Abbild des Gegenstandes ermitteln.

Das Linienmuster wird bei den bekannten Verfahren durch einen Blitzlichtprojektor mittels eines Diapositivs erzeugt. Nachteilig ist jedoch, daß bei der Ermittlung der Abmessung von großen Körpern, zum Beispiel der menschlichen Körpermaße, wo es erforderlich ist, zumindest den Rumpf darzustellen, alle Linien bis auf die Linien, die unter einem Winkel von 45° zur Horizontalen verlaufen mit einem Umrechnungsfaktor versehen werden müssen, um zu exakten Werten zu gelangen.

Der vorliegenden Erfindung liegt von daher die Aufgabe zugrunde, die bekannten Verfahren dahingehend zu verbessern, daß auf die Umrechnung verzichtet werden kann, ohne daß die erforderliche Genauigkeit der Meßergebnisse unterschritten wird.

Diese Aufgabe wird bei dem Verfahren der eingangs erwähnten Art dadurch gelöst, daß das Linienmuster mittels einer Vielzahl von Lichtquellen erzeugt wird, deren optische Achsen unter einem Winkel von nahezu 45° zur Vertikalen verlaufen. Durch die erfindungsgemäße Maßnahme ist die Abweichung der einzelnen außerhalb der optischen Achse liegenden Linien vom Einfallswinkel 45° so gering, daß keine nennenswerte Verfälschung der Ergebnisse auftritt.

Mit besonderem Vorteil ist der Abstand der einzelnen Linien zueinander zumindest der in der jeweiligen optischen Achse verlaufenden Linien zueinander gleich.

Die Erfindung betrifft weiterhin eine Vorrichtung zur Durchführung des Verfahrens, bestehend aus einer Kamera und einer das Linienmuster erzeugenden oberhalb der Kamera angeordneten Lichtquelle. Diese Vorrichtung zeichnet sich dadurch aus, daß die Lichtquelle aus einer Vielzahl von einzelnen Lichtquellen besteht, daß die Lichtquellen übereinander angeordnet sind und daß jeder Lichtquelle ein ein Linienmuster aufweisendes Diapositiv zugeordnet ist.

Zweckmäßigerweise sind die Lichtquellen Blitzlichtprojektoren und sind die Blitzlichtprojektoren an einem gemeinsamen Träger befestigt. Der Träger verläuft vorteilhafter weise geneigt zur Vertikalen. Die Blitzlichtprojektoren müssen zwischen dem Gegenstand und der Kamera oberhalb der Kameraachse angeordnet sein. Durch die Neigung des Trägers ist sichergestellt, daß trotz der relativ großen Ausdehnung der Blitzlichtprojektorenbatterie in vertikaler Richtung diese nicht auf der erzeugten Abbildung erscheinen.

Die Lichtquellen sind zweckmäßigerweise fest installiert, zum Beispiel an der Raumdecke befestigt. Die Maßrasterebene, d.h. der Platz an dem der zu vermessende Gegenstand angeordnet wird und die Kamera sind dagegen in horizontaler Richtung verschiebbar. Durch diese Maßnahme ergibt sich der Vorteil, daß mit einem Meßlinienblock von ca. 1 m Höhe große wie auch kleine Menschen in ganzer Rumpflänge vermessen werden können.

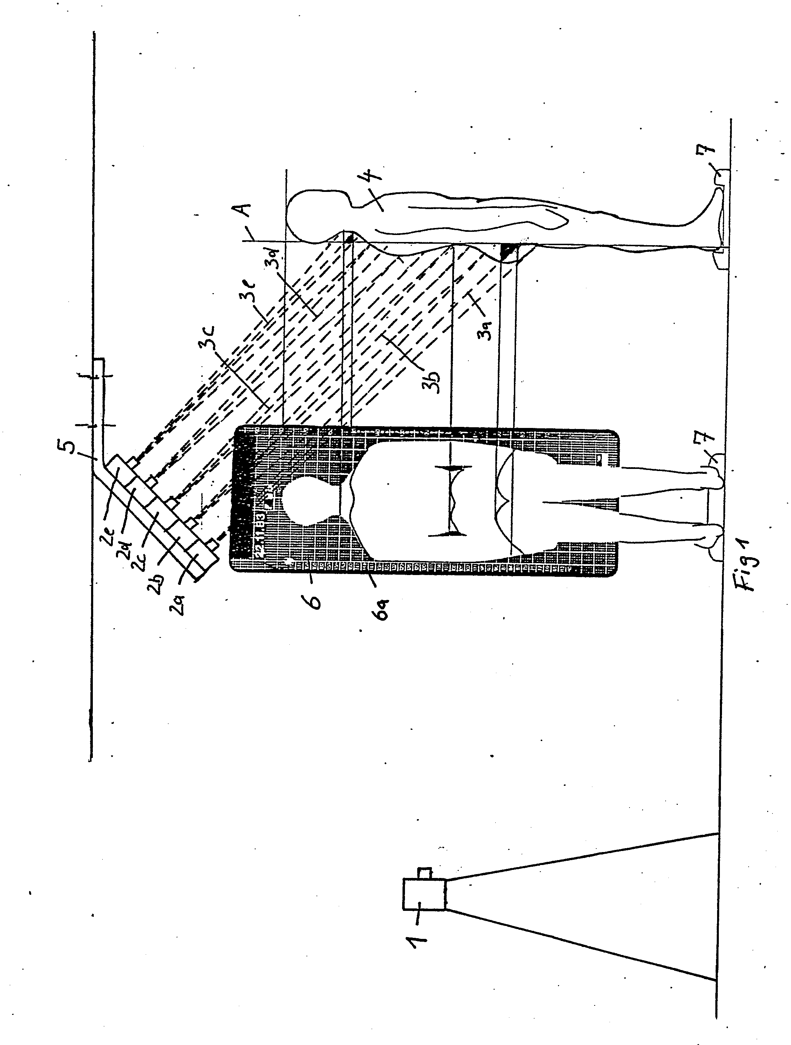

Die Erfindung ist anhand der in den Figuren 1 bis 3 schematisch dargestellten Ausführungsbeispiele näher erläutert.

Die Figur 1 zeigt eine seitliche Ansicht der erfindungsgemäßen Vorrichtung, wobei das erhaltende Abbild der zu vermessenden Figur nach links versetzt eingezeichnet ist.

Die Figur 2 zeigt den Strahlengang.

Die Figur 3 zeigt eine Draufsicht auf das Linienmuster eines Blitzlichtprojektors.

Mit 1 ist eine Kamera, beispielsweise eine Sofortbildkamera bezeichnet. Oberhalb der Kamera 1 sind die Blitzlichtprojektoren 2a,2b, 2c,2d und 2e angeordnet, die jeder mittels eines nicht näher dargestellten Diapositivs ein Linienbündel 3a, 3b, 3c, 3d, 3e auf eine zu vermessende Figur 4 projizieren. Die Blitzlichtprojektoren 2a - 2e sind an einem Träger 5 befestigt, der vorteilhafterweise an der Decke des Raumes befestigt ist, in dem die Vorrichtung aufgebaut ist. Der Teil des Trägers 5, an dem die Blitzlichtprojektoren 2a bis 2e befestigt sind, ist unter einem Winkel von vorzugsweise weniger als 90° zur Deckenebene abgewinkelt, so daß sein Ende bei niedrigen Raumhöhen nicht in den Strahlengang der Kamera 1 gelangt. Jeder einzelne Blitzlichtprojektor 2a bis 2e ist so an dem Träger 5 befestigt, daß die optische Achse jedes Blitzlichtprojektors 2a bis 2e exakt unter einem Winkel von 45° auf die Meßrasterebene A auftrifft. Die Genauigkeit der Messung ist umso höher, je mehr Linien auf die Meßrasterebene projiziert werden. Vorteilhaft ist ein Abstand der Linien von ca. 2 cm. Bei einer Meßhöhe für den Rumpf von in etwa l Meter ergeben sich somit 50 Linien. Bei der dargestellten Anzahl von fünf Blitzlichtprojektoren 2a bis 2e würden sich somit l0 Linien für jeden Blitzlichtprojektor 2a bis 2e ergeben. Die von der optischen Achse jedes Blitzlichtprojektors 2a bis 2e entfernt liegenden Linien fallen somit unter einem von 45° abweichenden Winkel auf die Meßrasterebene A, jedoch ist diese Abweichung so gering, daß die Abweichung in Kauf genommen werden kann.

Das in der Frontansicht erkennbare Meßraster 6 wird zweckmäßigerweise wie in der DE-OS 34 25 9l3 auf das Filmmaterial projiziert.

In der Meßrasterebene A befindet sich seitlich neben der Position des zu vermessenden Gegenstandes eine Meßlatte 6a, die Höhenmarkierungen und eine weiße Fläche aufweist, auf welche durch das projizierte Meßraster die Zahlen der fortlaufend nummerierten Linien dargestellt werden. Dadurch wird die Meßrasterebene A als Bezugsebene für die dreidimensionale Vermessung auf dem Photo dargestellt.

Damit ein zu vermessender Mensch stets in Ruhestellung vermessen werden kann, ist eine Balancewaage 7 vorgesehen, wie sie aus der DE-OS 33 0l 864 bekannt ist.

Auf der Frontansicht ist gut erkennbar, wie durch die Linien des Linienmusters Wölbungen dargestellt werden.

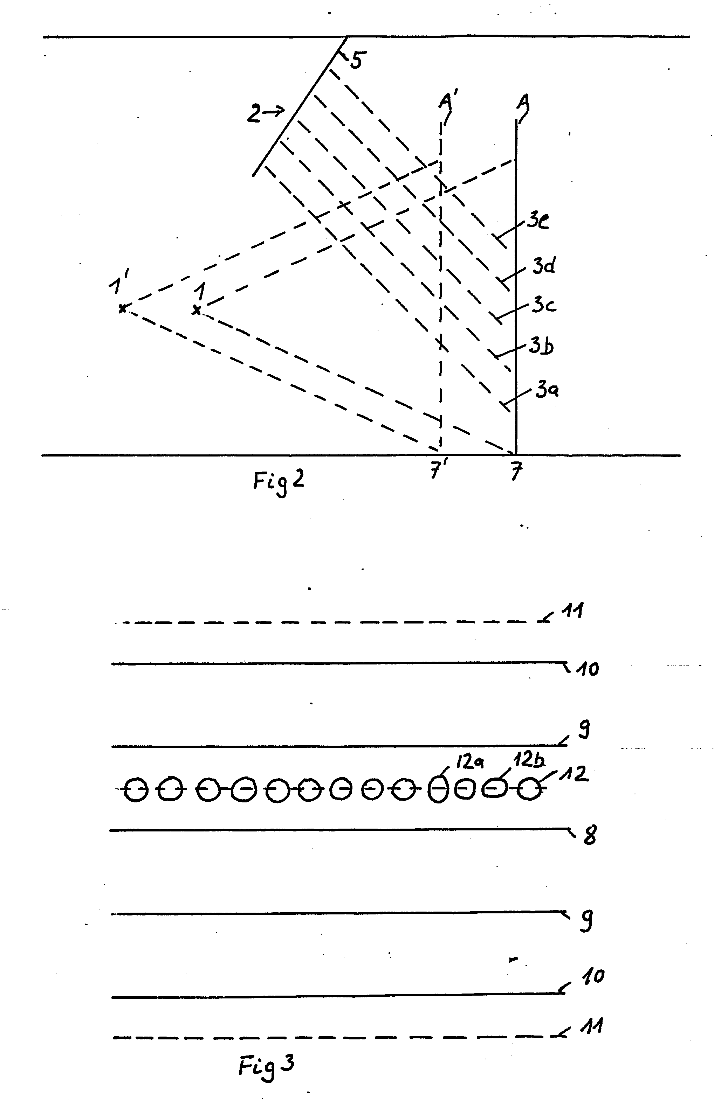

Die Figur 2 zeigt den Strahlengang der Kamera l sowie der Blitzlichtprojektorenbatterie 2. Durch Verschiebung der Kamera l und der Balancewaage 7 in horizontaler Richtung kann die Höhe des Linienmusters auf der Meßrasterebene verschoben werden. Dadurch können in besonders vorteilhafter Weise mit einem Meßlinienblock von in etwa l m Höhe große wie auch kleine Menschen in ganzer Rumpflänge vermessen werden.

Die Figur 3 zeigt das Linienmuster eines Blitzlichtprojektors 2a bis 2e, der fünf Linien projiziert, d.h. bei einer Abdeckung von l00 cm sind in diesem Fall bei einem Linienabstand von 2 cm zehn Blitzlichtprojektoren erforderlich. Die mittlere Linie 8 bildet die optische Achse. Die Abweichung der Linien 9 und l0 in Bezug auf den Winkel 45° ist so gering, daß diese Abweichung bei der Ermittlung des Meßergebnisses vernachlässigbar ist.

Die gestrichelt dargestellten Linien ll bilden die Überdeckungslinie zu dem benachbarten Linienmuster.

Zwischen den Linien 8, 9 und l0 kann noch in vorteilhafter Weise eine Reihe von Symbolen, zweckmäßigerweise Kreise l2 angeordnet sein, so daß leicht Deformationen auf der Oberfläche des zu vermessenden Körpers, auch zwischen den Linien, erkennbar werden. Die Kreise l2 werden bei einer vorhandenen Deformation elliptisch verformt. (s. l2a, l2b)

## Ansprüche

l. Verfahren zur Ermittlung der Abmessungen eines Gegenstandes auf photographischem Wege, insbesondere der menschlichen Körpermaße, bei dem eine Photographie des Gegenstandes zusammen mit einem Meßraster hergestellt und während der Aufnahme ein schräg von oben einfallendes Muster aus horizontal verlaufenden Linien auf den Gegenstand projiziert wird, dadurch gekennzeichnet, daß das Linienmuster mittels einer Vielzahl von Lichtquellen erzeugt wird, deren optische Achsen unter einem Winkel von nahezu 45° zur Vertikalen verlaufen.

2. Verfahren nach Anspruch l, dadurch gekennzeichnet , daß das Linienmuster mit gleichem Abstand der in der optischen Achse liegenden Linien zueinander projiziert wird.

3. Verfahren nach Anspruch l, dadurch gekennzeichnet, daß der Abstand aller Linien des Linienmusters gleich ist.

4. Vorrichtung zur Durchführung des Verfahrens nach einem oder mehreren der Ansprüche l bis 3, bestehend aus einer Kamera und einer das Linienmuster erzeugenden oberhalb der Kamera angeordneten Lichtquelle, dadurch gekennzeichnet, daß die Lichtquelle aus einer Vielzahl von einzelnen Lichtquellen besteht, daß die Lichtquellen übereinander angeordnet sind und daß jeder Lichtquelle ein ein Linienmuster aufweisendes Diapositiv zugeordnet ist.

5. Vorrichtung nach Anspruch 4, dadurch gekennzeichnet, daß die Lichtquellen Blitzlichtprojektoren sind und die Blitzlichtprojektoren an einem gemeinsamen Träger befestigt sind.

6. Vorrichtung nach Anspruch 5, dadurch gekennzeichnet, daß der Träger geneigt zur Vertikalen verläuft.

7. Vorrichtung nach einem oder mehreren der Ansprüche 4 bis 6, dadurch gekennzeichnet, daß die Lichtquellen fest installiert, die Photoebenen bzw. Meßrasterebene und die Kamera in horizontaler Richtung verschiebbar sind.

Fig 1

Fig 2

Fig 3

| | Europäisches Patentamt | **EUROPÄISCHER RECHERCHENBERICHT** | Nummer der Anmeldung |
| | | | EP 87 10 9284 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | DE-A-3 425 913 (U.M. LANDWEHR) * Zusammenfassung; Seite 6, Zeilen 3-17; Figur 1 * | 1,4,5 | A 61 B 5/10 G 01 B 11/24 G 01 C 11/00 |
| Y | EP-A-0 121 353 (NEW YORK INSTITUTE OF TECHNOLOGY) * Zusammenfassung; Seite 1, Zeilen 4-7; Seite 5, Zeile 29 - Seite 6, Zeile 13; Figur 3 * | 1,4,5 | |
| A | | 7 | |
| A | NOUVELLE REVUE D'OPTIQUE, Band 6, Nr. 2, März/April 1975, Seiten 67-86, Paris, FR; P. BENOIT et al.: "Characterization and control of threedimensional objects using fringe projection techniques" * Seiten 70-71, Abschnitt 3.1: "Principle"; Seiten 77-78, Abschnitt 4.3: "Real time contour line observation through a mask" * | 1-3 | |
| A | US-A-4 187 011 (P.L. DI MATTEO et al.) * Zusammenfassung; Spalte 3, Zeilen 6-31,59-62; Figur 1 * | 1,4 | |

**RECHERCHIERTE SACHGEBIETE (Int. Cl.4)**

A 61 B
G 01 B
G 01 C

Der vorliegende Recherchenbericht wurde für alle Patentansprüche erstellt.

| Recherchenort | Abschlußdatum der Recherche | Prüfer |
|---|---|---|
| DEN HAAG | 06-10-1987 | FERRIGNO, A. |